# EUROPEAN PATENT SPECIFICATION

(11) **EP 0 938 448 B1**
(45) Date of publication and mention of the grant of the patent: **25.07.2001**
(21) Application number: 97948817.8
(22) Date of filing: 27.10.1997
(51) Int. Cl.: C01B 23/00, A61K 33/00

(54) **PROCESS AND APPARATUS FOR PURIFYING AND RECOVERING XENON AND OTHER NOBLE GASES USED IN ANAESTHETIC SYSTEMS**
VERFAHREN UND VORRICHTUNG ZUR REINIGUNG UND GEWINNUNG VON XENON UND SONSTIGEN EDELGASEN VERWENDET IN NARKOSESYSTEMEN
PROCEDE ET APPAREIL SERVANT A PURIFIER ET A RECUPERER DU XENON ET D'AUTRES GAZ RARES UTILISES DANS DES SYSTEMES ANESTHESIQUES

(30) Priority: 29.10.1996 IT MI962238
(43) Date of publication of application: 01.09.1999
(73) Proprietor: SIAD SOCIETA' ITALIANA ACETILENE & DERIVATI S.p.A., 24126 Bergamo (IT)
(72) Inventor: BOSO, Luca, I-24126 Bergamo (IT)
(74) Representative: Minoja, Fabrizio, Dr.
(86) International application number: EP9705936
(87) International publication number: WO9818718

(56) References cited:
- DE-C- 4 411 533
- US-A- 4 874 592
- US-A- 5 039 500
- US-A- 5 099 834
- DATABASE WPI Section Ch, Week 9638 Derwent Publications Ltd., London, GB; Class B07, AN 96-382550 XP002059184 & RU 2 049 487 C (DOCTOR TRAINING CENTRAL INST) , 10 December 1995

## Description

The present invention relates to a process and the associated apparatus for purifying and recovering noble gases used in anaesthesia, in particular xenon.

### Background of the invention

Since 1940 noble gases have played a significant role in the development and characterization of theories on anaesthetic action. In particular xenon exhibits many properties of the ideal anaesthetic and is of particular interest because it is the only noble gas to act as an anaesthetic under conditions of standard atmospheric pressure. In this sense, xenon has been proposed as an improved substitute of the traditional anaesthetics by inhalation, such as nitrous oxide. However, xenon is very expensive and requires specific systems for its use. Therefore a system for its recovery is proposed, which allows its normal use in operating theatres.

Xenon is an inert gas and not very likely to participate in any chemical reaction when used as an anaesthetic and is moreover non-toxic, non-teratogenic, is probably not metabolized (unlike ethers, halothanes and fluoranes) and can be used for rapid induction of and recovery from anaesthesia.

Xenon also possesses other physicochemical and pharmacological properties favourable for anaesthetic action. Its solubility in different media can be expressed in the form of a distribution coefficient, which is defined as the ratio of the concentration of the agent in two phases at equilibrium. The blood/gas distribution coefficient is equal to 0.14 and is the major factor determining the ratio of induction of and recovery from anaesthesia. It is believed that in the case of nitrous oxide (an anaesthetic gas commonly used in the operating theatre), this coefficient is three times higher. In man, the MAC (minimum alveolar concentration) is 0.71.

Studies in animals and in man have been carried out without any evidence of major complications even after one hour of anaesthesia. Xenon has been found to be a potential agent for inducing analgesic and anaesthetic effects. In comparative studies with nitrous oxide, with both at a concentration of 70%, the total amount of fentanyl required by each patient is considered an objective indication of the power and efficacy of the two gases. Patients treated with xenon required 0.05 mg of fentanyl while patients treated with nitrous oxides received 0.24 mg thereof, thus showing the greater efficacy of xenon. Furthermore, it has been shown in clinical studies that xenon has no effects on the haemodynamic and cardiovascular systems and on the local organs, especially on the heart and the brain.

From an environmental point of view, xenon offers many advantages over other anaesthetics, insofar as it does not pose any problems or dangers for the environment, the operating theatre and the medical hospital staff.

Xenon is prepared by fractionation of air and other sources, which are particularly rich in this element. In air, it is present at very low concentrations of about 0.09 ppm by volume, while the degree of purity required for its application in anaesthesia varies from 99.9 to 99.999% by volume. The cost of xenon depends on its scarce presence and on the high purity required. For a surgical operation on a patient of 70 kg lasting one hour, a quantity of about 10 litres of xenon is consumed when using a closed circuit of 3 litres. This involves a much higher expense compared to the anaesthetics used today, which increases even more if an open circuit is used.

In conclusion, xenon has to be considered an ideal anaesthetic owing to its properties in terms of pharmacodynamics and safety, its efficacy is well documented, and in comparison with nitrous oxide it has shown greater efficacy without harmful effects.

As discussed above, the factor limiting its common use is its cost.

Marx T. et al. have described a system for recovering xenon directly from the exhaled air of the anaesthetized patient. The exhaled air is treated by means of a filtering system consisting of a cold trap, activated carbon and molecular sieves in order to remove volatile substances. The oxygen/nitrogen/xenon mixture leaving this filter is subsequently compressed to a pressure of 60 atmospheres and cooled to +16°C, converting the noble gas into the liquid state and separating it off from the gaseous impurities. The purified gas consists of a mixture of 80% xenon, 12% oxygen and 8% nitrogen. The authors conclude that an additional technique is necessary for separating and purifying xenon without, however, giving any indication as to how to carry out such a purification.

RU 2049487 discloses an apparatus for regeneration of xenon from gas-narcotic mixtures, comprising at least two adsorbers, a high-pressure cylinder for collection of gas. The adsorbers are in the form of metallic cups filled with sorbents, e.g. activated charcoal.

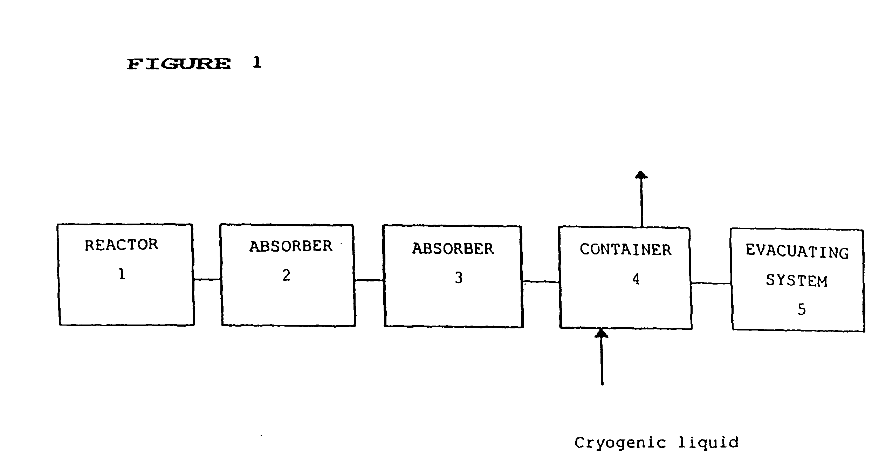

It has now been found, and this forms the subject-matter of the present invention, that xenon like other noble gases, such as argon and krypton, can be recovered and purified by removing its impurities using an apparatus such as illustrated in the figure.

### Summary of the invention

The present invention relates to an apparatus for purifying and recovering noble gases used in anaesthetic systems, which comprises essentially:
a) a catalytic reactor for oxidizing volatile hydrocarbons, carbon monoxide and other organic substances exhaled by the patient;
a') an optional absorber for water-soluble organic substances exhaled by the patient.
b) a water/carbon dioxide absorber;
c) a cryogenic container and, if desired,
d) a vacuum system for stripping off nitrogen and oxygen.

The present invention also relates to a process for recovering and purifying noble gases used in anaesthetic systems, which comprises essentially the steps of:
a) treating the exhaled air of the anaesthetized patient by means of a catalytic reactor for oxidizing hydrocarbons, carbon monoxide and other organic substances exhaled by the patient at a temperature above 200°C to give a first gaseous mixture which is substantially free of hydrocarbons and carbon monoxide;
a') optionally treating said first gaseous mixture obtained from step a) by means of absorbing water-soluble organic substances exhaled by the patient.
b) treating said gaseous mixture obtained in step a) by means of a water/carbon dioxide absorber to give a second gaseous mixture which is substantially anhydrous and substantially free of carbon dioxide;
c) treating said second mixture by means of a cryogenic container thermostatted at a temperature between - 100°C and -200°C to give a liquefied or solid mixture; and, if desired,
d) treating said liquefied or solidified mixture by means of an evacuating system for stripping off nitrogen and oxygen at a pressure between 0.1 and 300 mbar.

These and other subject-matters of the present invention will be illustrated in detail below by way of examples and figures, in which:
the figure illustrates schematically a possible embodiment of the apparatus according to the present invention, in which:
(1) is a catalytic reactor for oxidizing methane and other organic substances present (saturated and unsaturated hydrocarbons, carbon monoxide and organic substances present in the exhaled air);
(2) is an optional absorber for organic water-soluble substances exhaled by the patient, such as, for example, acetone;
(3) is a water/carbon dioxide absorber:
(4) is a cryogenic container thermostatted at a temperature between -100°C and -200°C and made of steel or else of an aluminium alloy or of glass, which is thermostatted with a liquid coolant, such as, for example, liquid nitrogen or else liquid oxygen or liquid argon or else liquid air;
(5) is an optional system for removing nitrogen and oxygen which is based on vacuum-stripping (using a mechanical pump or Venturi ejector or another equivalent system) of container (4).

### Detailed description of the invention

The present invention is applied to a treatment for purifying and recovering a noble gas from anaesthetic mixtures containing said gas or mixtures of noble gases. Said treatment can be applied to a patient, animal or man, subjected to anaesthesia.

In a first embodiment of the invention, the exhaled air of the patient can be treated directly by connecting the apparatus to the ventilation device or, if desired, the anaesthetic gas, in particular xenon, can be re-used during the course of the surgical operation, giving rise to a closed cycle or, alternatively, the purified anaesthetic gas can suitably be collected for its later use. If the treatment of the exhaled air takes place directly in the operating theatre, the apparatus of the present invention can be connected to the ventilation device using connecting lines in accordance with the specification of ISO 5356-1 or another equivalent standard and using a ventilator for transporting the gas at a flow rate of more than 100 ml/hour.

In a second embodiment of the present invention, the exhaled air of the patient under anaesthesia can be collected in suitable containers, for example in bags used for collecting gaseous samples or compressed in cylinders, and sent to a different location for treating and recovering the anaesthetic gas.

Alternatively, the exhaled air can be collected in suitable systems after any of steps a) - c) and subsequently treated starting from the step immediately following the collection step.

In a preferred embodiment of the invention, the anaesthetic gas is xenon.

According to a preferred embodiment of the present invention, the exhaled air to be treated is transferred to reactor (1), where an oxidative catalytic system is present for removing saturated and unsaturated hydrocarbons, preferably methane, and carbon monoxide. The catalytic reactor is maintained at a temperature above 200°C, preferably between 200 and 500°C, most preferably at 350°C. The catalyst consists of a noble metal, such as palladium or platinum, or an alloy thereof or a mixture of metals or alloys therof. It is also possible to use catalysts based on other metals performing this function, for example manganese, chromium, vanadium and alloys, mixtures and combinations thereof. The catalyst can be supported on alumina or any other suitable support known in the art, for example carbon. The catalyst may be in various forms, in the form of powder, granules or other customary embodiments. The reactor can be made of steel, aluminium or glass and its dimensions will depend on the amount of gas to be treated. The dimensions of the reactor are such as to reduce the presence of methane in the xenon to levels of less than 15 parts per million by volume, and saturated and unsaturated hydrocarbons, if present, to the same levels, while carbon monoxide is reduced below 0.1 part per million. In the present invention, "substantially free" is understood to mean those impurity levels below which the noble gas is acceptable for use in anaesthesia.

At the reactor exit, there is an optional absorber (2) for water-soluble organic substances exhaled by the patient, such as, for example, acetone, which consists of a concentrated alkaline solution (5 - 30% w/v) of potassium hydroxide or hydroxides of other alkaline metals, such as sodium, magnesium and calcium or combinations thereof. The dimensions of the absorber are such that acetone and, analogously, other organic substances soluble in an aqueous solution are reduced to levels below 5 ppm.

Xenon enters the subsequent reactor (3), where it is freed of the moisture and carbon dioxide present, if the latter was not already eliminated by the reactor (2). The dimensions of the absorber are such that the presence of moisture and carbon dioxide is reduced to levels below 100 ppm by means of an absorber based on molecular sieves, although alumina or zeolites can also be used.

In the container (4), freezing takes place by cooling with a cryogenic liquid to such a temperature that liquefaction or solidification of the noble gas, in particular xenon, can occur. This temperature is between -100°C and -200°C, and the cryogenic liquid can be nitrogen, or else argon, or else oxygen, or liquid air.

A further purification may become necessary, which is done by evacuating the container (4) with the aid of an evacuating system (5) using a mechanical vacuum pump or a Venturi type ejector having a degree of vacuum between 0.1 mbar and 300 mbar. This allows low-boiling gases, such as nitrogen, oxygen and argon, to be removed from the xenon or other noble gases. The degree of evacuation will be a function of the degree of vacuum of the pump and of the evacuation time so as to allow the gas to reach the specific characteristics utilizable for anaesthesia.

The various elements which constitute the apparatus comprise conventional connecting and distributing means for fluids, the configuration of which can be easily determined by the person skilled in the art. For example, it is possible to use tubes made of steel or of a plastic material whose internal diameter is between 1 and 30 mm and gas-collecting systems (gasometers) if the process is carried out in batch mode.

Once the xenon is recovered, it is re-usable for a new anaesthetic treatment after complete analysis of the impurities present. The obtainable degree of purity in this recovery system reaches 99.99% while the recovery rate can exceed 90%.

### EXAMPLE 1

The test for recovering xenon was carried out on the exhaled air of four patients who had been treated with xenon as an anaesthetic at the school specialized in anaesthesia and intensive care of the University Department of Surgery at the Hospital of Santa Chiara (Pisa).

25 litres of xenon present in the exhaled air of four patients were treated until completion of anaesthesia. The purification system used consisted of a 0.15 l catalytic reactor maintained at a temperature of 350°C and a 0.1 l absorber containing molecular sieves.

After being passed through the catalytic reactor and the purifying device, the anaesthetic gas was frozen in a 1 litre container cooled with liquid nitrogen to a temperature of -190°C. The subsequent evacuation with a mechanical vacuum pump up to a vacuum of 1 mbar made it possible to free the xenon of nitrogen and oxygen. At the end of the treatment, 23 litres of xenon corresponding to a recovery of 92% were obtained.

In the final gas, 100 ppm of methane, less than 0.1 ppm of carbon monoxide, less than 1 ppm of acetone, less than 1 ppm of nitrogen oxides and less than 100 ppm of water and carbon dioxide were found.

Moreover, the search for other impurities produced no results.

### EXAMPLE 2

### Compression test

4 PVF bags of about 20 litres in standard conditions were filled with the exhaled air of a patient. Analysis of each flask gave the following results:

| Conc. % | | | |
|---|---|---|---|
| Flask | Nitrogen | Oxygen | Xenon |
| JL2 | 63.1 | 22.2 | 14.7 |
| JL5 | 65.6 | 24.2 | 10.2 |
| JL6 | 64.4 | 28.2 | 7.4 |
| JL7 | 63.8 | 28.0 | 8.2 |

Subsequently, the whole was transferred to a PVC/polyester bag of 125 litres: the amount of xenon present, in terms of grammes, was 40 g.

This was followed by compressing the mixture using a 5 m³/hour compressor oil free, after which 32 g were found in the cylinder (80% recovery). A reason for the lower recovery is the not insignificant dead volume inside the compressor with these quantities.

The 1 litre cylinder, which at first was at atmospheric pressure, was found to be charged to 66 bar in standard conditions.

| Analysis of the cylinder: | |
|---|---|
| N₂ | 64.8% |
| O₂ | 25.1% |
| CO₂ | 0.7% |
| Xe | 8.7% |
| H₂O | 200 ppm (saturation) |

The amount of xenon is lower due to the above-mentioned problems of dead volume in the pump.

Finally, the xenon was purified with a recovery of 90%.

## Claims

1. Apparatus for purifying and recovering noble gases used in anaesthetic systems, which comprises essentially:
a) a catalytic reactor for oxidizing volatile hydrocarbons, carbon monoxide and other organic substances exhaled by the patient;
a') an optional absorber for water-soluble organic substances exhaled by the patient;
b) a water/carbon dioxide absorber;
c) a cryogenic container and, if desired,
d) a vacuum system for stripping off nitrogen and oxygen.

2. Apparatus according to Claim 1, in which between reactor a) and absorber b) an absorber a') for water-soluble organic substances exhaled by the patient is interposed.

3. Apparatus according to Claim 1, in which the catalytic reactor is suitable for oxidizing methane and, if present, other volatile saturated and unsaturated hydrocarbons.

4. Apparatus according to any one of Claims 1 or 3, in which said catalytic reactor a) is based on a metallic catalyst consisting of one or more metals or compounds thereof selected from the group consisting of manganese, chromium, vanadium, palladium, platinum or combinations thereof.

5. Apparatus according to Claim 4, in which the catalyst is supported on alumina or carbon.

6. Apparatus according to Claim 2, in which said absorber is a system comprising an aqueous solution containing 5 to 30% w/v of a hydroxide of an alkali metal or alkaline earth metal, preferably potassium hydroxide.

7. Apparatus according to Claim 6, in which the absorbed substance is acetone.

8. Apparatus according to any one of Claims 1 to 7, in which in said absorber b) the active element comprises an absorbing material selected from the group consisting of zeolites, molecular sieves and alumina.

9. Apparatus according to any one of Claims 1-8, in which in said cryogenic container c) the cryogenic liquid is selected from the group consisting of nitrogen, oxygen, air and argon.

10. Apparatus according to any one of Claims 1-9, in which in said vacuum system d) the vacuum is achieved by means of a mechanical pump or else by a Venturi system.

11. Apparatus according to any one of Claims 1-10, in which the noble gas is selected from the group consisting of argon, krypton and xenon or mixtures thereof, and is preferably xenon.

12. Apparatus according to any one of Claims 1-11, additionally comprising suitable connecting and distributing means for fluids.

13. Apparatus according to any one of Claims 1-12, additionally comprising means for recovering the exhaled air directly from the respiratory tract of the anaesthetized patient.

14. Apparatus according to any one of Claims 1-12, additionally comprising a means for connection to a system for collecting the exhaled air from the respiratory tract of the anaesthetized patient.

15. Process for recovering and purifying noble gases used in anaesthetic systems, essentially comprising the steps of:
a) treating the exhaled air of the anaesthetized patient by means of a catalytic reactor for oxidizing hydrocarbons, carbon monoxide and other organic substances exhaled by the patient at a temperature above 200°C to give a first gaseous mixture which is substantially free of hydrocarbons and carbon monoxide;
a') optionally treating said first gaseous mixture obtained from step a) by means of a step of absorbing water-soluble organic substances exhaled by the patient;
b) treating said gaseous mixture obtained in step a) by means of a water/carbon dioxide absorber to give a second gaseous mixture which is substantially anhydrous and substantially free of carbon dioxide;
c) treating said second mixture by means of a cryogenic container thermostatted at a temperature between-100°C and -200°C to give a liquefied or solid mixture; and, if desired,
d) treating the liquefied or solidified mixture by means of a vacuum system for stripping off nitrogen and oxygen at a pressure value between 0.1 and 300 mbar.

16. Process according to Claim 15, in which in step a) the temperature is between 200 and 500°C, preferably 350°C.

17. Process according to Claim 15, in which said first gaseous mixture obtained from step a) is treated by means of a step a') of absorbing water-soluble organic substances exhaled by the patient.

18. Process according to Claim 17, in which said absorbing step gives rise to a gaseous mixture which is substantially free of acetone.

19. Process according to Claim 15, in which step a) gives rise to said first gaseous mixture containing methane and hydrocarbons in an amount of less than 15 ppm v/v and carbon monoxide in an amount of less than 0.1 ppm v/v.

20. Process according to Claim 17, in which said absorption of organic substances is carried out until a residual value of less than 5 ppm v/v is reached.

21. Process according to Claim 15, in which step b) gives rise to a gaseous mixture containing water and carbon dioxide in an amount of less than 100 ppm v/v.

22. Process according to Claim 15 and any one of Claims 16-21, in which said purification is carried out directly on the exhaled air of the anaesthetized patient.

23. Process according to Claim 15 and any one of Claims 16-21, in which purification is carried out on the exhaled air of the anaesthetized patient previously collected in suitable collecting systems.

24. Process according to Claim 15 and any one of Claims 16-21, in which the exhaled air can be collected in suitable systems after any of steps a) - c) and is subsequently treated starting from the step immediately following the collecting step.

25. Process according to any one of Claims 15-24, in which said noble gas is selected from the group consisting of argon, krypton and xenon, or mixtures thereof, and is preferably xenon.

26. Use of the apparatus of Claims 1-14 in the process of Claims 15-25.

27. Use according to Claim 26, in which the exhaled air of the anaesthetized patient is introduced directly into said apparatus.

28. Use according to Claim 26, in which the exhaled air of the anaesthetized patient is first collected and then introduced into said apparatus.

29. Use according to Claim 28, in which the exhaled air of the anaesthetized patient is collected in suitable systems after any one of steps a) - c) and subsequently treated starting from the step immediately following the collecting step.

30. Use according to Claim 26, in which the exhaled air of the anesthetized patient is first collected, then compressed in cylinder and finally introduced in said apparatus.

## Patentansprüche

1. Vorrichtung zur Reinigung und Gewinnung von Edelgasen, die in Anästhesiesystemen verwendet werden, wobei die Vorrichtung im wesentlichen umfaßt:
a) einen katalytischen Reaktor zum Oxidieren flüchtiger Kohlenwasserstoffe, Kohlenmonoxid und anderen organischen Substanzen, die von dem Patienten ausgeatmet werden;
a') einen optionalen Absorber für wasserlösliche organische Substanzen, die von dem Patienten ausgeatmet werden;
b) einen Wasser-/Kohlendioxidabsorber;
c) einen Kryocontainer und, falls gewünscht,
d) ein Vakuumsystem zum Abtrennen von Stickstoff und Sauerstoff.

2. Vorrichtung gemäß Anspruch 1, bei der zwischen Reaktor a) und Absorber b) ein Absorber a') für wasserlösliche organische Substanzen angeordnet ist, die von dem Patienten ausgeatmet werden.

3. Vorrichtung gemäß Anspruch 1, bei der der katalytische Reaktor zur Oxidation von Methan geeignet ist und, wenn, vorliegend von anderen flüchtigen, gesättigten und ungesättigten Kohlenwasserstoffen.

4. Vorrichtung gemäß einem der Ansprüche 1 oder 3, bei der der katalytische Reaktor a) auf einem metallischen Katalysator basiert, der aus einem oder mehreren Metallen oder Verbindungen besteht, die aus der Gruppe bestehend aus Mangan, Chrom, Vanadium, Palladium, Platin oder Kombinationen davon ausgewählt ist.

5. Vorrichtung gemäß Anspruch 4, bei der der Katalysator auf einem Träger aus Aluminiumoxid oder Kohlenstoff angeordnet ist.

6. Vorrichtung gemäß Anspruch 2, bei der der Absorber ein System ist, das eine wässrige Lösung umfaßt, die 5 bis 30 % w/v eines Hydroxids eines Alkalimetalls oder eines Erdalkalimetalls umfaßt, bevorzugt Kaliumhydroxid.

7. Vorrichtung gemäß Anspruch 6, bei der die absorbierte Substanz Aceton ist.

8. Vorrichtung gemäß einem der Ansprüche 1 bis 7, bei der das aktive Element in dem Absorber b) ein absorbierendes Material umfaßt, das aus der aus Zeoliten, Molekularsieben und Aluminiumoxid bestehenden Gruppe ausgewählt ist.

9. Vorrichtung gemäß einem der Ansprüche 1 bis 8, bei der in dem Kryocontainer c) die Kryoflüssigkeit aus der aus Stickstoff, Sauerstoff, Luft und Argon bestehenden Gruppe ausgewählt ist.

10. Vorrichtung gemäß einem der Ansprüche 1 bis 9, bei der in dem Vakuumsystem d) das Vakuum mittels einer mechanischen Pumpe oder andernfalls durch ein Venturi-System erzielt wird.

11. Vorrichtung gemäß einem der Ansprüche 1 bis 10, bei der das Edelgas aus der aus Argon, Krypton und Xenon oder Mischungen davon bestehenden Gruppe ausgewählt und bevorzugt Xenon ist.

12. Vorrichtung gemäß einem der Ansprüche 1 bis 11, zusätzlich geeignete Verbindungs- und Verteilungsmittel für Fluide umfassend.

13. Vorrichtung gemäß einem der Ansprüche 1 bis 12, zusätzlich Mittel zur Gewinnung der ausgeatmeten Luft direkt aus dem Respirationstrakt des anästhetisierten Patienten umfassend.

14. Vorrichtung gemäß einem der Ansprüche 1 bis 12, zusätzlich ein Mittel zur Verbindung eines Systems zum Sammeln der ausgeatmeten Luft aus dem Respirationstrakt des anästhetisierten Patienten umfassend.

15. Verfahren zur Gewinnung und Reinigung von in Anästhesiesystemen verwendeten Edelgasen, im wesentlichen die Schritte umfassend:
a) Behandeln der ausgeatmeten Luft der anästhetisierten Patienten mittels eines Katalysereaktors zur Oxidation von Kohlenwasserstoffen, Kohlenmonoxid und anderen von dem Patienten ausgeatmeten Substanzen bei einer Temperatur oberhalb 200 °C, um eine erste gasförmige Mischung zu ergeben, die im wesentlichen frei von Kohlenwasserstoffen und Kohlenmonoxid ist;
a') optional Behandeln der ersten, aus Schritt a) erhaltenen gasförmigen Mischung mittels eines Schritts des Absorbierens von wasserlöslichen organischen Substanzen, die von dem Patienten ausgeatmet wurden;
b) Behandeln der in Schritt a) erhaltenen gasförmigen Mischung mittels eines Wasser-/Kohlendioxidabsorbers, um eine zweite gasförmige Mischung zu ergeben, die im wesentlichen wasserfrei und im wesentlichen frei von Kohlendioxid ist;
c) Behandeln der zweiten Mischung mittels eines auf eine Temperatur zwischen -100 °C und -200 °C thermostatierten Kryocontainers, um eine verflüssigte oder feste Mischung zu ergeben und, falls gewünscht,
d) Behandeln der verflüssigten oder verfestigten Mischung mittels eines Vakuumsystems, um Stickstoff und Sauerstoff bei einem Druck zwischen 0,1 und 100 mbar abzuziehen.

16. Verfahren gemäß Anspruch 15, bei dem in Schritt a) die Temperatur zwischen 200 und 500 °C ist, bevorzugt 350 °C.

17. Verfahren gemäß Anspruch 15, bei dem die erste aus Schritt a) erhaltene gasförmige Mischung mittels eines Schritts a') des Absorbierens von wasserlöslichen organischen Substanzen, die von dem Patienten ausgeatmet wurden, behandelt wird,

18. Verfahren gemäß Anspruch 17, bei dem der Absorptionsschritt eine gasförmige Mischung ergibt, die im wesentlichen frei von Aceton ist.

19. Verfahren gemäß Anspruch 15, bei dem Schritt a) eine erste gasförmige Mischung liefert, die Methan und Kohlenwasserstoffe in einer Menge von weniger als 15 ppm v/v und Kohlenmonoxid in einer Menge von weniger als 0,1 ppm v/v enthält.

20. Verfahren gemäß Anspruch 17, bei dem die Absorption organischer Substanzen durchgeführt wird, bis ein Restwert von weniger als 5 ppm v/v erreicht ist.

21. Verfahren gemäß Anspruch 15, bei dem Schritt b) eine gasförmige Mischung liefert, die Wasser und Kohlendioxid in einer Menge von weniger als 100 ppm v/v enthält.

22. Verfahren gemäß Anspruch 15 und einem der Ansprüche 16 bis 21, bei dem die Reinigung direkt an der ausgeatmeten Luft des anästhetisierten Patienten durchgeführt wird.

23. Verfahren gemäß Anspruch 15 und einem der Ansprüche 16 bis 21, bei dem die Reinigung an der zuvor in geeigneten Sammelsystemen gesammelten, ausgeatmeten Luft des anästhetisierten Patienten durchgeführt wird.

24. Verfahren gemäß Anspruch 15 und einem der Ansprüche 16 bis 21, bei dem die ausgeatmete Luft nach irgendeinem der Schritte a) bis c) in geeigneten Systemen gesammelt werden kann und nachfolgend, ausgehend von dem unmittelbar auf den Sammlungsschritt folgenden Schritt behandelt wird.

25. Verfahren gemäß einem der Ansprüche 15 bis 24, bei dem das Edelgas aus der aus Argon, Krypton und Xenon oder Mischungen davon bestehenden Gruppe ausgewählt und bevorzugt Xenon ist.

26. Verwendung der Vorrichtung gemäß einem der Ansprüche 1 bis 14 in dem Verfahren gemäß einem der Ansprüche 15 bis 25.

27. Verwendung gemäß Anspruch 26, bei dem die ausgeatmete Luft des anästhetisierten Patienten direkt in die Vorrichtung eingeführt wird.

28. Verwendung gemäß Anspruch 26, bei der die ausgeatmete Luft des anästhetisierten Patienten zuerst gesammelt und nachfolgend ind ie Vorrichtung eingeführt wird.

29. Verwendung gemäß Anspruch 28, bei der die ausgeatmete Luft des anästhetisierten Patienten in geeigneten Systemen nach einem der beliebigen der Schritte a) bis c) gesammelt und nachfolgend, ausgehend von dem unmittelbar auf den Sammlungsschritt folgenden Schritt behandelt wird.

30. Verwendung gemäß Anspruch 26, bei der die ausgeatmete Luft des anästhetisierten Patienten zuerst gesammelt, nachfolgend in einem Zylinder komprimiert und schließlich in die Vorrichtung eingeführt wird.

## Revendications

1. Dispositif destiné à purifier et récupérer les gaz rares utilisés dans des systèmes d'anesthésie qui comprend essentiellement :
a) un réacteur catalytique destiné à oxyder les hydrocarbures volatils, le monoxyde de carbone ainsi que d'autres substances organiques expirées par le patient ;
a') un absorbeur facultatif destiné aux substances organiques solubles dans l'eau expirées par le patient ;
b) un absorbeur d'eau/dioxyde de carbone ;
c) un récipient cryogénique et, si on le souhaite.
d) un système de vide destiné à enlever l'azote et l'oxygène.

2. Dispositif selon la revendication 1, dans lequel entre le réacteur a) et l'absorbeur b) un absorbeur a') destiné aux substances organiques solubles expirées par le patient est interposé.

3. Dispositif selon la revendication 1, dans lequel le réacteur catalytique est approprié pour oxyder le méthane et, s'ils sont présents, d'autres hydrocarbures volatils saturés et insaturés.

4. Dispositif selon l'une quelconque des revendications 1 ou 3, dans lequel ledit réacteur catalytique a) est fondé sur un catalyseur métallique constitué d'un ou plusieurs métaux ou de composés de ceux-ci choisis parmi le groupe constitué du manganèse, du chrome, du vanadium, du palladium, du platine ou de combinaisons de ceux-ci.

5. Dispositif selon la revendication 4, dans lequel le catalyseur est supporté sur de l'alumine ou du carbone.

6. Dispositif selon la revendication 2, dans lequel ledit absorbeur est un système comprenant une solution aqueuse contenant 5 à 30 % poids/volume d'un hydroxyde d'un métal alcalin ou d'un métal alcalino-terreux, de préférence l'hydroxyde de potassium.

7. Dispositif selon la revendication 6, dans lequel la substance absorbée est l'acétone.

8. Dispositif selon l'une quelconque des revendications 1 à 7, dans lequel, dans ledit absorbeur b) l'élément actif comprend un matériau absorbant choisi parmi le groupe constitué de zéolites, de tamis moléculaires et d'alumine.

9. Dispositif selon l'une quelconque des revendications 1 à 8, dans lequel, dans ledit récipient cryogénique c) le liquide cryogénique est choisi parmi le groupe constitué de l'azote, de l'oxygène, de l'air et de l'argon.

10. Dispositif selon l'une quelconque des revendications 1 à 9, dans lequel, dans ledit système de vide d) ledit vide est obtenu au moyen d'une pompe mécanique ou par un système de Venturi.

11. Dispositif selon l'une quelconque des revendications 1 à 10, dans lequel le gaz rare est choisi parmi le groupe constitué de l'argon, du krypton et du xénon ou de mélanges de ceux-ci, et est de préférence le xénon.

12. Dispositif selon l'une quelconque des revendications 1 à 11, comprenant en outre des moyens appropriés de raccordement et de distribution destinés à des fluides.

13. Dispositif selon l'une quelconque des revendications 1 à 12, comprenant en outre un moyen destiné à récupérer l'air expiré directement depuis les voies respiratoires du patient anesthésié.

14. Dispositif selon l'une quelconque des revendications 1 à 12, comprenant en outre un moyen pour le raccordement à un système destiné à recueillir l'air expiré depuis les voies respiratoires du patient anesthésié.

15. Procédé de récupération et de purification de gaz rares utilisés dans des systèmes anesthésiques, comprenant essentiellement les étapes consistant à :
a) traiter l'air expiré du patient anesthésié au moyen d'un réacteur catalytique destiné à oxyder les hydrocarbures, le monoxyde de carbone ainsi que d'autres substances organiques expirées par le patient à une température au-dessus de 200 °C pour donner un premier mélange gazeux qui est pratiquement exempt d'hydrocarbures et de monoxyde de carbone,
a') traiter facultativement ledit premier mélange gazeux obtenu à partir de l'étape a) au moyen d'une étape consistant à absorber les substances organiques solubles dans l'eau expirées par le patient,
b) traiter ledit mélange gazeux à l'étape a) au moyen d'un absorbeur d'eau/dioxyde de carbone pour donner un second mélange gazeux qui est pratiquement anhydre et pratiquement exempt de dioxyde de carbone,
c) traiter ledit second mélange au moyen d'un récipient cryogénique thermostaté à une température entre -100 °C et -200 °C pour donner un mélange liquéfié ou solide, et, si on le souhaite,
d) traiter le mélange liquéfié ou solidifié au moyen d'un système de vide destiné à enlever l'azote et l'oxygène à une valeur de pression entre 0,1 et 300 mbar.

16. Procédé selon la revendication 15, dans lequel à l'étape a) la température se situe entre 200 et 500 °C, de préférence 350 °C.

17. Procédé selon la revendication 15, dans lequel ledit premier mélange gazeux obtenu à partir de l'étape a) est traité au moyen d'une étape a') consistant à absorber les substances organiques solubles dans l'eau expirées par le patient.

18. Procédé selon la revendication 17, dans lequel ladite étape d'absorption donne naissance à un mélange gazeux qui est pratiquement exempt d'acétone.

19. Procédé selon la revendication 15, dans lequel l'étape a) donne naissance audit premier mélange gazeux contenant du méthane et des hydrocarbures en une proportion de moins de 15 ppm v/v et du monoxyde de carbone en une proportion de moins de 0,1 ppm v/v.

20. Procédé selon la revendication 17, dans lequel ladite absorption de substances organiques est exécutée jusqu'à ce qu'une valeur résiduelle de moins de 5 ppm v/v soit atteinte.

21. Procédé selon la revendication 15, dans lequel l'étape b) donne naissance à un mélange gazeux contenant de l'eau et du dioxyde de carbone en une proportion de moins de 100 ppm v/v.

22. Procédé selon la revendication 15 et l'une quelconque des revendications 16 à 21, dans lequel ladite purification est exécutée directement sur l'air expiré du patient anesthésié.

23. Procédé selon la revendication 15 et l'une quelconque des revendications 16 à 21, dans lequel la purification est exécutée sur l'air expiré du patient anesthésié préalablement recueilli dans des systèmes de recueil appropriés.

24. Procédé selon la revendication 15 et l'une quelconque des revendications 16 à 21, dans lequel l'air expiré peut être recueilli dans des systèmes appropriés après l'une quelconque des étapes a) à c) et est ensuite traité en partant de l'étape qui suit immédiatement l'étape de recueil.

25. Procédé selon l'une quelconque des revendications 15 à 24, dans lequel ledit gaz rare est choisi parmi le groupe constitué de l'argon, du krypton et du xénon, ou de mélanges de ceux-ci, et est de préférence le xénon.

26. Utilisation du dispositif selon les revendications 11 à 14 dans le procédé des revendications 15 à 25.

27. Utilisation selon la revendication 26, dans laquelle l'air expiré du patient anesthésié est introduit directement dans ledit dispositif.

28. Utilisation selon la revendication 26, dans laquelle l'air expiré du patient anesthésié est tout d'abord recueilli et est ensuite introduit dans ledit dispositif.

29. Utilisation selon la revendication 28, dans laquelle l'air expiré du patient anesthésié est recueilli dans des systèmes appropriés après l'une quelconque des étapes a) à c) et est ensuite traité en partant de l'étape qui suit immédiatement l'étape de recueil.

30. Utilisation selon la revendication 26, dans laquelle l'air expiré du patient anesthésié est tout d'abord recueilli, puis comprimé dans un cylindre et enfin introduit dans ledit dispositif.
